Europäisches Patentamt

European Patent Office

Office européen des brevets

(11) Publication number: **0 435 463 A1**

## EUROPEAN PATENT APPLICATION

(21) Application number: 90312918.7

(22) Date of filing: 28.11.90

(51) Int. Cl.⁵: **A61F 5/02**, A61F 5/01

The title of the invention has been amended (Guidelines for Examination in the EPO, A-III, 7.3).

(30) Priority: 28.11.89 NZ 231564

(43) Date of publication of application:
03.07.91 Bulletin 91/27

(84) Designated Contracting States:
AT BE DE DK FR GB IT LU NL SE

(71) Applicant: McKenzie, Robin Anthony
"Woodhill", Smithfield Road
Waikanae(NZ)

Applicant: McKenzie, Joy Lynette
"Woodhill", Smithfield Road
Waikanae(NZ)

(72) Inventor: McKenzie, Robin Anthony
"Woodhill", Smithfield Road
Waikanae(NZ)
Inventor: McKenzie, Joy Lynette
"Woodhill", Smithfield Road
Waikanae(NZ)

(74) Representative: Boyes, Kenneth Aubrey et al
Frank B. Dehn & Co. Imperial House 15-19
Kingsway
London WC2B 6UZ(GB)

(54) Orthopaedic lumbar support.

(57) This invention provides an orthopaedic support to maintain lumbar lordosis. The support is in the form of an elongate pad (4) that is substantially D-shaped in cross-section and that has been moulded from material that has a compressibility such that a 25 percent indentation into the pad requires from about 8.8 kPA to about 10.7 kPA applied pressure. The surface of the pad (8) that in use engages a person's back is slightly concave along its length. In use the pad is placed between the low back and the back rest of a seat.

EP 0 435 463 A1

# AN ORTHOPAEDIC SUPPORT

This invention relates to an orthopaedic support which is suitable for assisting a person to maintain lumbar lordosis when sitting on a seat that has a back rest. The support is particularly useful in treatment programs for the treatment, rehabilitation and prevention of back pain in humans.

In humans the spine is held in a more or less vertical position while the person is sitting or standing. In this position, the lumbar spine bears the compressive weight of the body above it and transmits this weight to the pelvis. Therefore the lumbar spine provides a flexible connection between the upper and lower halves of the body and protects the spinal cord. In order to bear the weight of the upper body, the spinal column has developed a series of curves that allow for better shock absorption and flexibility.

One of the curves is an inward curve in the small of the back just above the pelvis. The hollow in the back created by this curve is called the lumbar lordosis. When a person stands upright, the required lordosis is usually present but is lost whenever the lower back is rounded. This usually occurs when the person is sitting or bending forwards and is accentuated if the spine is subjected to vibrations as would occur in a vehicle. If the lordosis is lost sufficiently frequently and for long periods, problems in the lower back develop. These problems and, the remedial effects of obtaining and maintaining correct lordosis, have been documented in the books by Robin A McKenzie called "The Lumbar Spine: Mechanical Diagnosis and Therapy", Spinal Publications Limited, Waikanae, New Zealand 1981 and "Treat your own back", Spinal Publications Limited, Waikanae, New Zealand 1985. These books supports studies by Andersson et al; 1975; "The Sitting Postures: An Electromyographic and Discometric Study", Orthopaedic Clinics of North America, 6 1; 105 to 120.

When a person is sitting in a seat that has a back rest, the person is unlikely to maintain lordosis for any length of time since it requires constant attention and constant effort to maintain lordosis. Therefore it is usually necessary to place some form of support between the back of the chair and the lower back. One support known to the applicant is the "lumbar roll" disclosed on pages 24 and 25 of the book "Treat your own back" supra. The lumbar roll consist of a cylindrical pad of foamed polyurethane of diameter of about 12 cm. The lumbar roll is produced by forming a large slab of polyurethane foam and then cutting a large number of cylindrical pads from the slab. Each cylindrical pad is then covered by a durable and aesthetically pleasing cover.

In use the lumbar roll is positioned between the back rest of a chair and the lower portion of the person's back. The lumbar roll has proved to be extremely successful and is used in treatment programs throughout the world.

There are however a number of problems with the lumbar rolls. First, it has been found that the lumbar rolls do not maintain their mechanical properties. After about 18 months of constant use, it is found that the roll becomes too compressible and no longer provides adequate support.

It has also been found that the lumbar rolls are not sufficiently adaptable in use such that a standard sized roll can be used by any individual within a range of widely varying sizes and weights. In particular it has been found that larger, heavier people do not obtain sufficient support whilst smaller, lighter people find the roll large and uncomfortable to use.

Another problem is that the lumbar roll is prone to roll away once the user gets up from the seat or ceases to maintain backward pressure on the roll. This problem has been solved to some extent in the art by the provision of rolls which are hemispherical or D-shaped in cross-section. These rolls still have the other disadvantages of the lumbar rolls.

Accordingly it is an object of this invention to provide an orthopaedic support that is suitable for inducing lordosis and which has relatively wide application and relatively good durability.

This invention provides an orthopaedic support comprising a moulded, compressible, elongate pad having a back engaging surface that is slightly concave in longitudinal direction and is of a length sufficient to extend substantially across a user's back and a width in the range of about 7 cm to about 15 cm. The pad has a seat engaging surface opposite the back engaging surface that has a substantially linear profile transverse its length when viewed at any point along its length. The pad has a thickness between its back engaging surface and its seat engaging surface in the range of 5.0 cm to 7.0 cm and the compressibility of the material making up the pad is such that a 25 percent indentation into the back engaging surface towards the seat engaging surface requires from 8.8 kPA to 10.7kPA applied pressure.

Preferably the pad is made from foamed polyurethane. Also the pad is preferably covered by a durable and aesthetically pleasing cover.

The pad is preferably substantially D-shaped in cross-section and the length of the pad may be in the range of 40 cm to 42 cm and the width in the range of 14 cm to 15 cm.

The seat engaging surface may be slightly convex in longitudinal direction and the ends of the pad may be tapered so that the pad is substantially ellipse-shaped in plan.

Preferably the compressibility of the material making up the pad is such that a pressure of 9.7 kPA applied to the back engaging surface towards the seat engaging surface creates a 25% indentation. A compressibility of about this amount results in a 1.5 cm to 2.5 cm compression in the thickness of the pad when the pad is used.

The use of a material of the required compressibility provides the significant advantage that the pad may be used by a large number of individuals within a range of widely varying sizes, weights and depths of adipose or fat covering. It is surprisingly the particular compressibility (or density) of the pad which makes the pad effective in all these conditions, particularly when combined with the specific thickness of the pad.

Equally surprisingly the particular contours of the pad also strongly influence the effectiveness of the pad over a broad range of seating conditions and individual body sizes.

An embodiment of the invention is described, by way of example only, with reference to the drawings in which:

Figure 1 is a side view of the orthopaedic support;

Figure 2 is a rear view of the orthopaedic support;

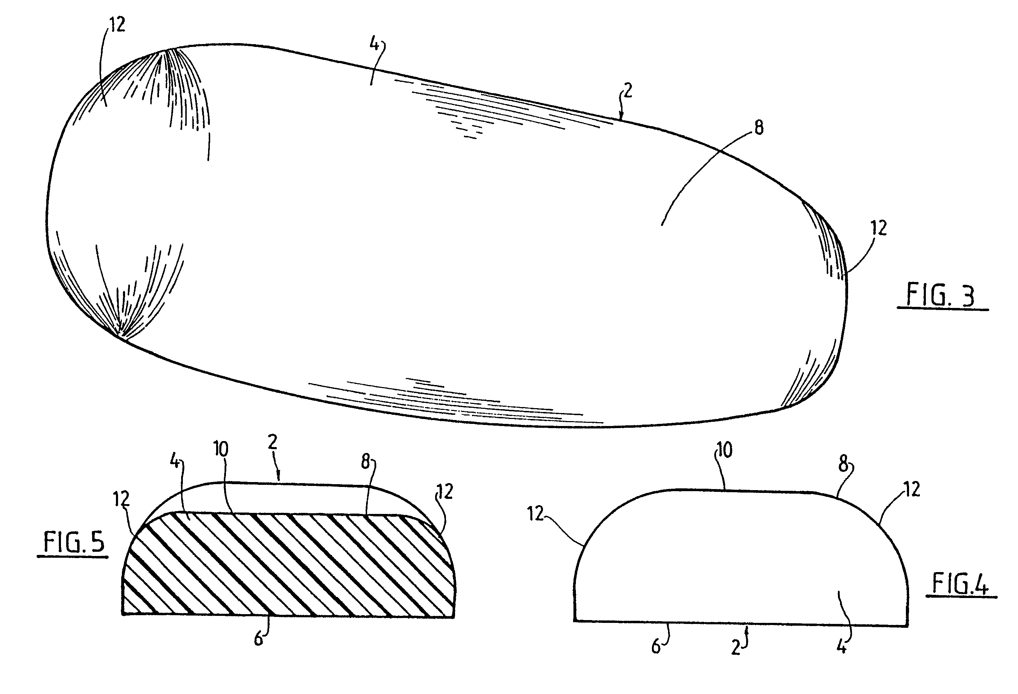

Figure 3 is a perspective, front view of the orthopaedic support;

Figure 4 is an end view of the orthopaedic support; and

Figure 5 is a cross section taken along A-A of figure 1.

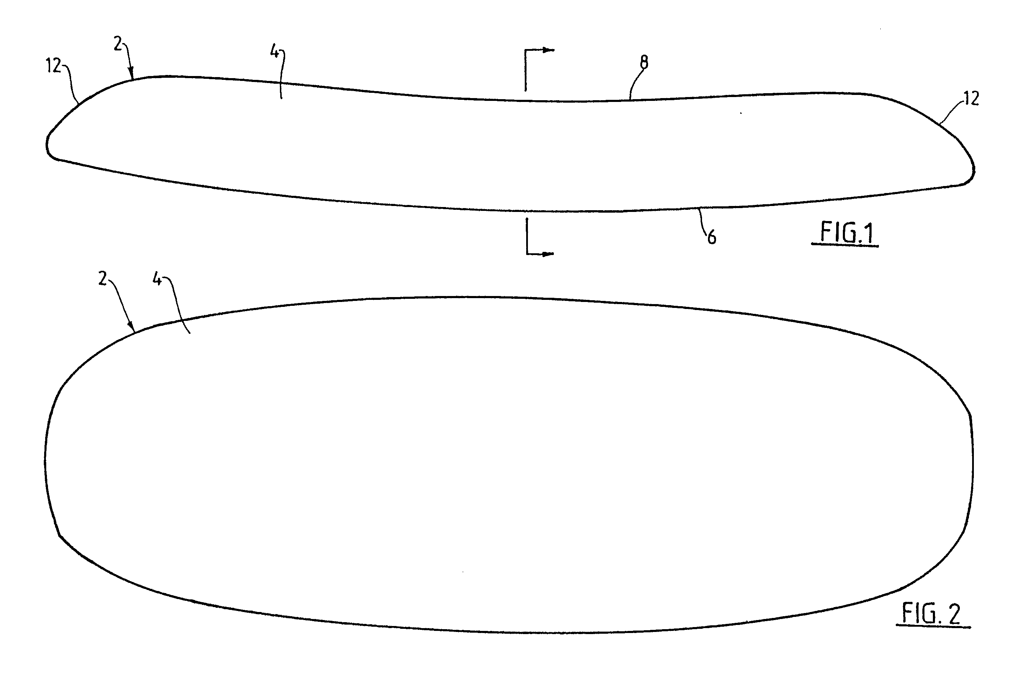

Referring to the drawings, the orthopaedic support 2 comprises a foamed polyurethane pad 4 which, when viewed in plan, is substantially ellipse shaped (as best illustrated in figure 2). The pad 4 has a seat-engaging surface 6 which is substantially linear transverse its length but which has a slight arcuate profile longitudinally to give it a slightly convex contour. This is best illustrated in figure 1.

The pad 4 has a back-engaging surface 8 which includes a central portion 10 which is substantially linear transverse its length. Arcuate portions 12 curve rearwardly from each side of the central portion 10 to meet the seat-engaging surface 6. The back-engaging surface 8 is slightly concave in longitudinal profile as best illustrated in figure 1.

The thickness of the pad between the seat-engaging surface 6 and the central portion 10 of the back-engaging surface 8 is substantially constant for the entire planar portion 10 and is about 5.5 cm. The pad 4 has a length of about 41 cm and a width of about 14.5 cm.

The foamed pad is compressible to an extent of about 2 cm when subjected to normal pressures. The terms "normal pressures" mean the pressures usually exerted on the pad by an average person's back.

The pad 4 is covered by a durable and flexible covering material. This material may include retaining means, such as velcro strips, to hold the pad 4 in position on the back of a chair or seat. In fact, the pad 4 also could form part of a truss or corset or other form of suitable clothing when continuous use is required.

The pad 4 may also be provided with straps (not shown) that are adjustable in length so that the pad 4 can be secured to the seat back. These straps can be secured to the covering material or may wrapped around the pad 4.

The pad 4 can be manufactured in a conventional foam machine such as the "Cannon C7" machine manufactured by Afros s.p.a. of Milan, Italy. Reagents conventionally used to provide polyurethane foam are used. For example the isocyanate component may be "Supracet VM 28" which is marketed by ICI Limited. The polyester component may be "Voranol CP 3913" which is marketed by the Dow Corporation. Of course any suitable reagents may be used.

The reagents are mixed in the machine and expelled through a nozzle into the lower portion of a mould. Once the lower portion of the mould has filled, the upper portion of the mould is closed on the lower portion and the reagents allowed to react to produce the polyurethane polymer. The foaming of the reagents in the closed mould creates sufficient pressure for a foam of the required compressibility to be obtained. The mould is shaped to provide the pad without the need for cutting of the foam.

To select the correct compressibility, the compressibility of the foam was measured using a standard indentation-hardness test at 25% deflection. Testing equipment such as that marketed by Firma Zwick of West Germany may be used. In these tests the moulded foam is held in position and a foot of selected size is pushed against the foam. The load required to achieve 25% deflection is then measured.

A 63 mm diameter foot was used in all tests and the required compressibility was achieved when loads in the range 2.8 kg or 3.4 kg resulted in 25% indentation. Particularly preferred were foams which required about 3.1 kg load to achieve 25% compression. Foams of this compressibility are obtained when the process described above is used but can be obtained using other processes known in the art; for example by injection moulding.

In practice, the most beneficial location of the orthopaedic support is on or just above the waist line. When used constantly the support will assist in maintaining lordosis.

Experiments were conducted in New Zealand by supplying orthopaedic supports of the shape illustrated in the drawings but of varying densities to patients in New Zealand. In all cases the patients reported that the support which required a 3.1 kg load in a 25% indentation-hardness test using a 63 mm diameter foot was superior. The patients reported that pains of postural origin decreased quicker with this support and that the support was more comfortable to use. It is believed that in use the support reduces pelvic rotation while the user is sitting and preserves lordosis. This reduces tension from the posterior anulus, disc pressure and myoelectric activity of the posterior paraspinal muscles. In all cases tested, back and leg pain was reduced and pain centralised in the low back.

It has also been found that the moulded support is much more durable than the lumbar rolls which were cut from polyurethane foam slabs. Testing has shown that after prolonged periods of constant use, the orthopaedic support suffers much less deterioration of its mechanical properties than the lumbar roll.

It will be appreciated that numerous modifications may be made to the embodiment described in this specification without departing from the scope of the invention as set out in the claims and interpreted by the description.

## Claims

1. An orthopaedic support comprising a compressible, elongate pad having a back engaging surface that is of a length sufficient to extend substantially across a user's back and a seat engaging surface opposite the back engaging surface, the pad having a width in the range of 7 cm to 15 cm, characterised in that the pad is moulded and has a thickness between its back engaging surface and its seat engaging surface in the range of 5.0 cm to 7.0 cm, the back engaging surface is slightly concave in longitudinal direction, the seat engaging surface has a substantially linear profile when the pad is viewed in cross-section at any point along its length, and the compressibility of the material making up the pad is such that a 25 percent indentation into the back engaging surface towards the seat engaging surface requires from 8.8 kPA to 10.7kPA applied pressure

2. An orthopaedic support according to claim 1 in which the material making up the pad is foamed polyurethane.

3. An orthopaedic support according to claim 1 or claim 2 in which the pad is substantially D-shaped in cross-section.

4. An orthopaedic support according to any of claims 1 to 3 in which the length of the pad is in the range of 40 cm to 42 cm and the width of the pad is the range of 14 cm to 15 cm.

5. An orthopaedic support according to any of claims 1 to 4 in which the seat engaging surface is slightly convex in longitudinal direction.

6. An orthopaedic support according to any of claims 1 to 5 in which the pad is covered by a durable cover.

7. An orthopaedic support according to any preceding claim wherein the compressibility of the material making up the pad is such that a 25 percent indentation into the back engaging surface towards the seat engaging surface requires 9.7 kPA applied pressure.

FIG.1

FIG.2

EP 0 435 463 A1

FIG. 3

FIG.4

FIG.5

## European Patent Office

# EUROPEAN SEARCH REPORT

Application Number

**EP 90 31 2918**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 796 315 (R.E. CREW) <br> * Claims; figures * <br><br> – – – | 1-7 | A 61 F 5/02 <br> A 61 F 5/01 |
| A | US-A-4 597 386 (M.I. GOLDSTEIN) <br> * Claims; figures * <br><br> – – – | 1-7 | |
| A | US-A-4 616 639 (W.C. HUBER) <br><br> – – – | | |
| A | US-A-3 765 721 (B.C. WATKIN) <br><br> – – – – – | | |

**TECHNICAL FIELDS SEARCHED (Int. Cl.5)**

A 61 F

The present search report has been drawn up for all claims

| Place of search | Date of completion of search | Examiner |
|---|---|---|
| The Hague | 28 February 91 | VILLENEUVE J-M.R.J. |